# EUROPEAN PATENT SPECIFICATION

(11) **EP 2 324 840 B1**
(45) Date of publication and mention of the grant of the patent: **20.03.2019**
(21) Application number: 09425472.9
(22) Date of filing: 18.11.2009
(51) Int. Cl.: A61K 36/28, A61P 1/16, A61P 25/00, A61P 31/04, A61P 31/10, A61P 31/16

(54) **Production of caffeoylquinic acids from plant cell cultures of echinacea angustifolia**
Herstellung von Kaffeoylchinasäuren aus Pflanzenzellkulturen aus Echinacea angustifolia
Production d'acides cafféoylquiniques à partir de cultures de cellules végétales d'Echinacea angustifolia

(43) Date of publication of application: 25.05.2011
(73) Proprietor: Croda Italiana S.p.A., 27036 Mortara (PV) (IT)
(72) Inventor: Sgaravatti, Elena, 36077 Altavilla Vicentina Vicenza (IT); Dal Toso, Roberto, 36077 Altavilla Vicentina Vicenza (IT); Pressi, Giovanna, 36077 Altavilla Vicentina Vicenza (IT)
(74) Representative: de Saint Viance, Isabelle Marie Fanny

(56) References cited:
- EP-A1- 1 671 535
- EP-A1- 2 319 914
- WO-A1-98/42188
- WO-A2-02/47703
- MAGGINI R ET AL: "Effect of post-harvest handling and extraction on the content of echinacoside and cynarin in the root tissues of Echinacea angustifolia" FOOD, AGRICULTURE & ENVIRONMENT, WFL, HELSINKI, vol. 8, no. 2, 1 January 2010 (2010-01-01) , pages 266-271, XP009137591 ISSN: 1459-0255
- WU CHUN-HUA ET AL: "Optimization of culturing conditions for the production of biomass and phenolics from adventitious roots of Echinacea angustifolia" JOURNAL OF PLANT BIOLOGY, vol. 49, no. 3, June 2006 (2006-06), pages 193-199, XP002596974 ISSN: 1226-9239
- CHUN-HUA WU ET AL: "Large-scale cultivation of adventitious roots of Echinacea purpurea in airlift bioreactors for the production of chichoric acid, chlorogenic acid and caftaric acid" BIOTECHNOLOGY LETTERS, SPRINGER NETHERLANDS LNKD- DOI:10.1007/S10529-007-9399-1, vol. 29, no. 8, 23 June 2007 (2007-06-23), pages 1179-1182, XP019523939 ISSN: 1573-6776
- PELLATI FEDERICA ET AL: "Analysis of phenolic compounds and radical scavenging activity of Echinacea spp." JOURNAL OF PHARMACEUTICAL AND BIOMEDICAL ANALYSIS, vol. 35, no. 2, 16 April 2004 (2004-04-16) , pages 289-301, XP002596975 & 10TH INTERNATIONAL MEETING ON RECENT DEVELOPMENTS IN PHARMACEUTICAL ANALYSIS (RDPA 2003; COGNE, AOSTA VALLEY, ITALY; JUNE 28-JULY 01, 2003 ISSN: 0731-7085

## Description

### Field of the invention

The present invention refers to extracts containing caffeoylquinic acids, in particular mono-, di-, and tri-caffeoylquinic acids, to their production and use in human and veterinary therapies, for nutritional and cosmetic purposes. Said extracts are obtained from "in vitro" cell cultures of cells belonging to the plant *Echinacea angustifolia.* In addition, the present invention concerns the preparation and use of said extracts for the production of drugs or nutritional or cosmetic substances, said extracts having multiple pharmacological properties.

### State of the art

Caffeoylquinic acids are polyphenolic molecules belonging to the wide family of secondary metabolites. During the last few years particular attention has been paid to these bioactive plant compounds because of their multiple biological activities. Biological activities of mono-, di-, and tri-caffeoylquinic acids have been largely described in the literature. In particular, these compounds are involved in the protection of proteins, lipids, and nucleic acids from free radical-induced damages (Gebhardt R. et al, 1997, Toxicol. Appl. Pharm. 144:279-286; Perez Garcia F. et al, 2000, Free Radical Res., 33: 661-665); they inhibit cholesterol biosynthesis, contribute to the prevention of atherosclerosis and vascular disorders (Brown J.E. Et al, 1998, Free Radical Res., 29:247-255; Kraft K., 1997, 4: 369-378; Pittlern MH et al, Perfusion, 11:338-340), show hepatoprotective, coleretic, and diuretic activities (Dogan S. et al, 2005, J. Agric. Food Chem., 53:776-785), antiviral activity aganinst HIV (Mcdougall B. et al, 1998, Antimicrob. Agents Ch., 42: 140-146; Shanina J. et al, 2001, Tetrahedron Lett., 42:3383-3385), antibacterial and antifungal activitities (Martinov et al, 1999, Acta Horticulturae, 501:111-114; Zhu XF, 2005, Fitoterapia; 76: 108-111), and potential tumor-preventive activity (Kurata R. et al, 2007, J. Agric. Food Chem. 55(1):185-190).

The presence of caffeoylquinic acids in plant tissues is subjected to wide fluctuations depending on geographical origin, season variability, and plant contamination with parasites. In addition, the plant content of caffeoylquinic acids is always very limited. Using stabilized and selected plant cell cultures is a valid alternative to obtain extracts with a high content of caffeoylquinic acids in industrial quantities.

Plant cell culture technology allows to overcome the problems mentioned above.

Some examples of caffoylquinic acids isolation from plant cell cultures are reported in the literature. Konczak I. et al. (2004, Journal of Biomedicine and Biotechnology, 5:287-292) isolated 4,5-dicaffeoylquinic acid, 3,5-dicaffeoylquinic acid, 3,4-dicaffeoylquinic acid, and 3,4,5-tricaffeoylquinic acid from cell cultures of sweet potatoes (Solanaceae family). Tanura H. et al. (2006, Mol. Nutr. Food Res., 50:396-400) demonstrated the presence of 3,4,5-tricaffeoylquinic acid in lettuce cell cultures.

The originality of the present invention depends on the preparation process of extracts with a high content of caffeoylquinic acids obtained from *Echinacea angustifolia* selected cell cultures.

### Summary of the invention

An object of the present invention is a process according to claim 1, for the production, also in industrial quantities, of extracts with a high content of caffeoylquinic acids from *Echinacea angustifolia*
cell cultures.

Further objects of the present invention are said extracts, according to claim 6, in particular for use in the preparation of drugs, nutritional, cosmetic, and zootechnical substances.

Further objects and advantages of the present invention will become more apparent from the following detailed description of the invention.

### Brief description of the figures

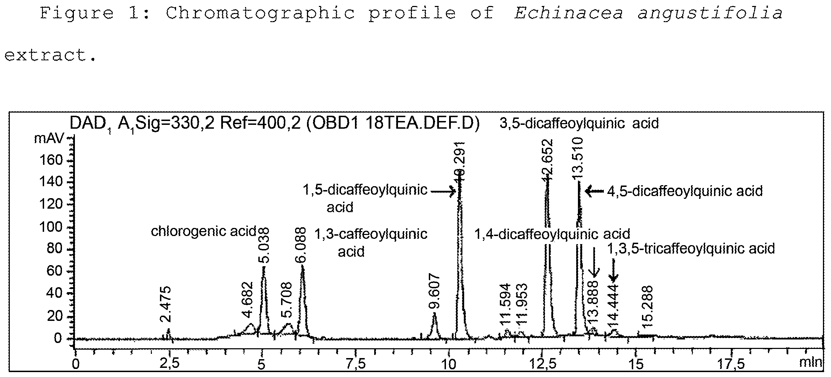

Figure 1 is an HPLC chromatogram of the *Echinacea angustifolia* extract.

### Detailed description of the invention

Caffeoylquinic acids isolated from *Echinacea angustifolia* cell culture are reported in the following Table 1.

**Table 1**

| ***Caffeoylquinic acids isolated from an Echinacea angustifolia selected cell culture*** |
|---|
| 3-caffeoylquinic acid |
| 1,3-dicaffeoylquinic acid |
| 1,4-dicaffeoylquinic acid |
| 1,5-dicaffeoylquinic acid |
| 4,5-dicaffeoylquinic acid |
| 3,5-dicaffeoylquinic acid |
| 1,3,5-tricaffeoylquinic acid |

The preparation process of extracts with a high content of caffeoylquinic acids, also in industrial quantities, envisages extraction from a biomass of *Echinacea angustifolia* selected cell cultures.

The process of the invention comprises obtaining calluses from tissues of *Echinacea angustifolia* plants, stabilizing the cell cultures obtained from callus tissues, selecting cultures with a high content of caffeoylquinic acids (obtained by standard methods reported in the literature: Schmauder H.P., Doebel P., Acta Biotechnolo., 1990; 10: 501-516; or as it will be described below), transferring the stabilized and selected cell cultures into liquid culture media, and producing high quantities of biomass in appropriate fermenters.

Hereinafter, an extraction was performed from said selected plant cell cultures using the process described below.

### Process for obtaining Echinacea angustifolia selected cell cultures.

This process envisages collecting tissues from plants of *Echinacea angustifolia,* the cleaning thereof, for example under running water, cutting into fragments of 2-5 cm, and sterilization on plates, for example by sequential treatment with 70% ethanol for approximately 15 minutes, 2% sodium hypochlorite for approximately 5 minutes, and finally 0.05% HgCl₂ for approximately 1 minute. Between treatments, the plant fragments are washed, typically three or more times, with sterile distilled water.

Each fragment of said tissue, chopped-up even further (explants), is placed on a Petri dish containing a nutrient medium, solidified by the addition of agar and supplemented with growth hormones without the addition of any antibiotics. The number of explants performed influences the outcome of the subsequent stages. Generally, from 2000 to 5000 uncontaminated explants are sufficient to proceed to the subsequent selection stage.

After a suitable period of time, an undifferentiated callus tissue forms, which is then multiplied after a transfer onto a greater surface area with fresh medium.

Furthermore, the plant cell culture derived from the undifferentiated callus tissue is stabilized preferably by means of a certain number of transfers (sub-cultures) onto fresh culture medium. Particularly, it has been observed that in order to obtain a stable cell culture, it is important to perform at least ten sub-cultures. Such medium is solid in nature and may be advantageously constituted by 0.8-1% agar in a standard culture medium, to which plant peptone has been added, allowing a balanced supply of aminoacids and guaranteeing the maintenance of good cell wall integrity. Preferably, plant peptone will be added in quantities ranging between 500 and 4000 mg/l of culture medium.

A "stable cell culture" is defined as a culture having the following characteristics:
- high and constant proliferation rate over time;
- preservation of the same phenotypic characteristics (cell colour, aggregate friability, size etc.) throughout various sub-cultures;
- constant content of secondary metabolite per unit of mass, over the course of the various sub-cultures (the content of the secondary metabolite is assessed by chemical analysis of the extracts);
- constant primary metabolite content (proteins, lipids, and polysaccharides) per unit of mass.

Following the stabilization stage, the cell culture is subjected to "clonal selection". Such selection consists of growing the stabilized cells for an appropriate amount of time (typically, 10-15 days of culture). Subsequently, individual cell aggregates are taken from the solid culture medium and each of said cell aggregates is inoculated into the liquid culture medium described above.

Following fermentation for a sufficient period of time to obtain adequate multiplication of the cell aggregate (hereinafter referred to as "clone"), a time generally comprised of between 10 and 15 days, the content of the metabolite of interest is determined for each clone.

Such operations may be repeated until a plant cell culture clone is selected, in which production of the metabolite of interest is the highest.

It should be remembered that alternating periods of culture on solid and liquid media is essential for the clonal selection process of the present invention. Hence, it is essential that the clonal selection process described above does not conclude with the identification of the most active clone, but is constantly repeated so as to keep the selected clone phenotypically homogeneous.

The selected plant cell culture is then multiplied in order to obtain a sufficient quantity of biomass to carry out the production fermentation stage. Said quantity will depend on the specific production requirements, on the kind of the plant cell culture used, and on the type of metabolite that it is desired to produce.

The biomass thus obtained may be passed directly into the final fermenter or can be subjected to one or more further growth stages in liquid medium, working with intermediate volumes.

Preferably, the process just illustrated comprises the stages of:
a) cultivating a predetermined plant cell culture, stabilized for a sufficient period of time to allow multiplication of said cell culture to give substantially distinct cell clusters, on solid medium;
b) removing said substantially distinct cell clusters from said solid medium and placing each of them in a separate liquid culture medium;
c) cultivating each of the said substantially distinct cell clusters in said liquid culture medium for a sufficiently long period of time to allow multiplication of said cell cluster and analytical determination of the primary and/or secondary metabolites produced thereby;
d) performing a qualitative and quantitative determination of the primary and/or secondary metabolites produced by each of said cell clusters in said liquid culture medium;
e) selecting the cell cluster capable of producing the greatest quantity of said metabolite of interest;
f) repeating the process cycle according to stages a), b), c), d), and e) on said cell cluster, selected according to stage e), a sufficient number of times until the quantity of said metabolite of interest produced by a selected cell cluster, and by the cell cluster deriving from further selection cycles, is essentially constant.

In addition, the subsequent fermentation may preferably comprise the following stages:
A) inoculation of said plant clone into liquid medium and the multiplication thereof for a sufficient period of time to obtain an increase in cellular mass of at least 300% of the weight thereof;
B) optionally, transfer of the suspension obtained from stage A) into fresh liquid culture medium and the multiplication thereof for a sufficient period of time to obtain an increase in cellular mass of at least 300% of the weight thereof;
C) optionally, repetition of stage B) at least one additional time;
D) transfer of the suspension obtained in stages A), B), or C) into fresh liquid culture medium in a fermenter to give a biomass and conduct the fermentation under such conditions and for a sufficient period of time so as to obtain, within said biomass, the production of at least one metabolite of interest;
E) extraction of at least one said metabolite of interest from said biomass.

In accordance with one preferred embodiment, the fermentation will normally be performed at a temperature between 15°C and 35°C, typically around 25°C and for a period of time normally between 7 and 40 days, preferably between 14 and 21 days. It is essential that the biomass be adequately aerated and that at the same time be kept stirred by means of stirring external to the fermenter. Indeed, it has been observed that the plant biomass is comprised of cells having cell walls that are poorly resistant to rupture. A stirrer submerged into the biomass acts mechanically on the cells and can easily cause the lysis thereof. However, it is necessary that the stirring, although delicate, be efficient, above all in the final fermentation stages when the biomass greatly increases in density. For the purposes of the present invention, particularly appropriate means of stirring are orbital means of stirring. Such means of stirring preferably operate at 40-200 rpm, more preferably at about 120 rpm.

It is appropriate that the volume of the container (fermenter) in which the fermentation occurs be considerably greater than the biomass volume. Typically, the volume of the reactor will be from 50% to 200% greater than the biomass volume.

As already mentioned, efficient fermentation requires adequate oxygenation. Oxygenation is normally performed by using sterile air with a flow rate of 0.5-4 1/minute, more preferably 2-2.5 1/minute, for a volume of 10 litres of biomass. Alternatively, gas mixtures containing from 10% to 100% v/v of oxygen may be used.

As mentioned previously in relation to stirring, also oxygenation by means of exceedingly violent bubbling can cause rupturing of the cell walls. Hence, it is necessary to ensure that oxygenation is performed delicately, for example by bubbling through appropriate diffusers. It will be preferable to use means of air or oxygen diffusion with nozzle delivery flow speeds comprised of between 10 m/min and 600 m/min, more preferably between 50 m/min and 350 m/min.

Also the shape of the fermentation chamber has significant importance. Indeed, it is recommended that it has a smooth and uniform surface, i.e. that there are no edges, corners, or other parts which can cause the rupture of the biomass cell walls.

According to one particular embodiment of the present invention, additives increasing water oxygen solubility will be added to the biomass. Such additives will be preferably selected from those substances defined as "artificial blood", for example perfluorinated hydrocarbons (PFC).

In particular, for the purposes of the present invention, stabilized cell cultures obtained from tissues of *Echinacea angustifolia* plants were selected for their ability to produce mono-, di-, and tri-caffeoylquinic acids in appropriate qualitative and quantitative proportions.

As a non-limiting example, conditions to prepare extracts from *Echinacea angustifolia* selected cell cultures will be described.

### Morphological characteristics of the Echinacea angustifolia cell line

The *Echinacea angustifolia* cell line denominated IRBEA66, DSMZ deposit number (Deutsche Sammlung von Mikroorganismen und Zellkulturen, Braunschweig, D): DSM 17522, deposit date: May 18^{th}, 2005, has a dark yellow color with few purple pigmentations and a friable appearance.

### Extraction process

The extraction process from cultures selected according to the invention comprises the stages of: i) forming a homogenized suspension of said cell culture, ii) eliminating particles, iii) extracting the resulting solution, at pH values ranging between 3 and 4, with a solvent capable of dissolving caffeoylquinic acids, or iv) extracting the resulting solution with hydrophobic interaction resins, at pH values between 3 and 4.

In particular, the extraction process from cell cultures comprises the following stages in sequence:
a) filtration of the biomass obtained from fermentation of *Echinacea angustifolia* cell cultures in order to recover cells only and discard the medium;
b) addition of an equal volume of a water-soluble alcohol, preferably ethanol (or any other water-soluble 2- to 5-carbon atoms alcohol), together with 0.1-0.8 g/l of ascorbic acid, preferably 0.5 g/l, to the cells;
c) complete homogenization, for example using an UltraTurrax or any other instrument capable of disgregating cells and their internal structures;
d) removal of cell debris, for example by filtration or centrifugation, preferably using a filtrating device such as a nylon or cotton mesh with a porosity between 10 and 300 µm, preferably between 50 and 150 µm;
e) removal of ethanol and part of water from the filtrate hydroalcoholic phase, for example by reduced pressure distillation;
f) pH adjustment to values between 3 and 4, for example by addition of 1.0-3.0 g/l, preferably 2.0 g/l, of citric acid or any other organic or mineral acid;
g) extraction of the suspension with an organic solvent in which caffeoylquinic acids are soluble, for example ethyl acetate or any other partially water-soluble solvent;
h) separation and anhydrification of the organic phase, for example using sodium sulphate or any other anhydrifying agent;
i) distillation at reduced pressure of the solvent from the anhydrous organic solution to give a solid compound with a total titer of caffeoylquinic acids higher than 10% by weight, preferably between 25% and 85% by weight.

As an alternative, a second preparation process can be used to obtain extracts with a total titer of caffeoylquinic acids higher than 10% by weight, preferably between 25% and 85% by weight, comprising:
a) homogenizing the entire culture, for example using an UltraTurrax or any other instrument capable of disgregating cells and their internal structures, after adjusting pH to about 8, for example by addition of 2 g/l of sodium bicarbonate or any other basic compound such as potassium bicarbonate or sodium carbonate;
b) removing particles from the suspension, for example by centrifugation or filtration using a filtration device such as a nylon or cotton mesh with a porosity ranging between 10 and 300 µm, more preferably between 50 and 150 µm;
c) reducing the filtrate pH to about 4, for example by addition of citric acid or any other organic or inorganic acid;
d) extracting the acidified solution with a solvent capable of dissolving caffeoylquinic acids, for example ethyl acetate or any other partially water-soluble solvent;
e) anhydrifying the organic phase, for example using sodium sulphate or any other anhydrifying agent, and distillating the organic phase, for example by reduced pressure filtration, to give a solid compound.

The operations described in g), h), and i) can be replaced by extraction from the acidic aqueous phase obtained in c) using hydrophobic interactions resins, such as those having a styrene or divinylbenzene matrix, preferably XAD4. Such resin can be eluted with an ethanol or methanol solution at a concentration varying between 50% and 95%, preferably 90%.

The eluate can be distilled at reduced pressure until ethanol is completely removed and the aqueous residue can be lyophilized and extracted with ethyl acetate.

The described processes allow to obtain extracts from *Echinacea angustifolia* selected cell cultures with a total titer of caffeoylquinic acids higher than 10% and preferably ranging between 25% and 85% by weight, and in particular extracts containing 3-caffeoylquinic acid and one or more acids selected among 1,3-dicaffeoylquinic acid, 1,4-dicaffeoylquinic acid, 1,5-dicaffeoylquinic acid, 3,5-dicaffeoylquinic acid, 4,5-dicaffeoylquinic acid, and 1,3,5-tricaffeoylquinic acid, preferably with a titer in relation to 3-caffeoylquinic acid higher than 10% by weight.

As a non-limiting example, examples of preparation of extracts with a high titer of caffeoylquinic acids from *Echinacea angustifolia* selected cell cultures are reported.

### EXAMPLE 1

### PREPARATION ON INDUSTRIAL BASIS OF EXTRACTS WITH A HIGH CONTENT OF CAFFEOYLQUINIC ACIDS FROM ECHINACEA ANGUSTIFOLIA CELL CULTURES

The preparation of extracts with a high content of caffeoylquinic acids from *Echinacea angustifolia* IRBEA66 cell cultures is reported as an example for the purposes of non-limiting illustration.

Calluses from the *Echinacea angustifolia* cell line IRBEA66 grown on solid medium (GAMBORG B5 containing 1% agar, with the addition of 20 g/l sucrose, 1 g/l plant peptone, 1 mg/l kinetin, 1 mg/l naphthaleneacetic acid, 0.2 mg/l indolacetic acid, pH 6.5) and subjected to sub-cultures for at least three months are inoculated in 10 x 300 ml flasks, each containing 50 ml of liquid medium (GAMBORG B5 added with 20 g/1 sucrose, 1 g/1 plant peptone, 1 mg/1 kinetin, 1 mg/1 naphthaleneacetic acid, 0.2 mg/1 indolacetic acid, pH 6.5) After 7 days of fermentation, cultures are used in turn as inoculum for 10 x 1000 ml flasks, each containing 250 ml of liquid medium. After 7 days of fermentation, 1000 ml flasks, each containing 250 ml of culture medium, are used in turn as inoculum for 10 x 3 1 flasks, each containing 1 1 of liquid medium. The suspensions obtained after 7 days of fermentation are used for inoculation into 2 x 100 1 fermenters. Fermentation is monitored by taking samples sequentially over time and concluded at day 14.

Solid sodium bicarbonate is added to the suspension until pH is adjusted to about 8, then the suspension is homogenized using an UltraTurrax and filtered through a nylon mesh with a porosity of 100 pm or centrifuged.

The aqueous solution is extracted according to the procedure described in claim 1.

A grey solid compound (718 g) containing 366 g of caffeoylquinic acids is obtained from the extraction process. The extract thus obtained contains 51% of caffeoylquinic acids.

### HPLC analysis

High pressure liquid chromatography (HPLC) is performed on a C18(2) Phenomenex 4.6 x 150 mm, 3 µm column using the following eluting mixtures:
Phase A: 0.1M citric acid buffer solution pH 4.2 + 0.2M Na₂HPO₄, diluted 1/20 in H₂O.
Phase B: 0.1M citric acid buffer solution pH 4.2 + 0.2M Na₂HPO₄, diluted 1/4 in H₂O, then diluted 1/5 in a CH₃CN:MeOH (1:1) mixture.

Flow rate: 0.8 ml/min
The used gradient is as follows:

| **T (min)** | **Phase B (%)** |
|---|---|
| 0 | 12 |
| 10 | 33 |
| 15 | 33 |
| 20 | 12 |

Quantification of compounds was performed at 330 nm.

**Table 2 - Qualitative and quantitative profile of caffeoylquinic acids contained in Echinacea angustifolia extract**

| **Product** | **RT (min)** | **Amount of product in the total extract (%)** |
|---|---|---|
| 3-caffeoylquinic acid | 4.90-5.0 | 29 |
| 1,3-dicaffeoylquinic acid | 6.0 | 4 |
| 1,4-dicaffeoylquinic acid | 9.6 | 5 |
| 1,5-dicaffeoylquinic acid | 10.2-10.3 | 2 |
| 3,5-dicaffeoylquinic acid | 12.65 | 6 |
| 4,5-dicaffeoylquinic acid | 13.5 | 5 |
| 1,3,5-tricaffeoylquinic acid | 14.44 | 3 |

Figure 1, enclosed herein, is a chromatogram of the *Echinacea angustifolia* extract measured at 330 nm.

As far as the multiple biological functions exerted by caffeoylquinic acids are concerned (widely described in the literature, as discussed above), the extracts obtained from the *Echinacea angustifolia* cell line of the invention can be advantageously used in pharmaceutical and cosmetic applications and in the human and zootechnical nutritional sectors.

The extracts of the invention can be advantageously used for the following pharmacological properties which they contain:
- anti-inflammatory activity, in the prevention and treatment of pathologies related to chronic and acute inflammatory states;
- cytoprotective and detoxifying activities;
- hepatoprotective activity;
- stimulation of biliary flow (coleretic effect)
- antibacterial activity, in the prevention and treatment of pathological disorders related to epidermal and cutaneous lesions derived from burns, incisions or lesions and mechanical traumas, diabetic sores, bedsores, thus facilitating cicatrization and tissue repair processes;
- antifungal activity;
- hypocholesterolizing activity;
- antiviral activity (in particular against HIV-1 virus);
- neuroprotective activity;
- antioxidant activity, in the prevention and treatment of pathological disorders related to free radical-induced damage such as: pathologies of the nervous systems with an anoxic, traumatic, and inflammatory component, degenerative pathologies of the nervous system also associated with aging or with an autoimmune and inflammatory component, reperfusion damages and cardiovascular disorders, atherosclerosis and muscular damage, dismetabolic disorders such as diabetes mellitus and the neurological, vascular, and ophtalmic complications thereof, toxic disorders such as alcoholism and the neurological complications thereof, besides hepatic cirrhosis, peripheral neuropathies including toxic neuropathies, cutaneous pathologies, including cellulitis and alopecia, mucosal pathologies including oropharyngeal, gastrointestinal and vaginal mucosae, dental pathologies, disorders of the respiratory apparatus, articular, tumoral, and ophthalmic pathologies, disorders of the auditory apparatus, and reduced immune response.

Doses, times, and routes of administration of the treatment will be selected on the basis of the type, stage, severity, and location of manifestation of the pathology. For all the pathologies mentioned above, systemic and oral administrations are indicated, but also topical and transdermal, and in any case so as to make active ingredients maximally available. For oral formulations, administrations in the form of tablets, lozenges, and capsules, but also powders and suspensions, are preferred; for topical treatment, gels, creams, ointments, and solutions compatible with dermal and mucosal use are preferred, in addition to eye washes for administration into the conjunctival sac. Injectable forms are formulated with solvents for pharmacological use compatible with intravenous, intramuscular, and subcutaneous administration.

The composition therapeutic dose varies, depending on the patient's age, weight, sex, and pathology, between 0.1 mg and 2 g per day and preferably between 5 and 150 mg per day, taken in a single dose or in 2-4 doses or in slow release forms, depending on the patient's therapeutic needs, and for periods of time varying between 1 and 120 days.

At appropriate concentrations, the same compositions may be formulated in the form of supplements, to be taken orally for prevention or as adjuvant treatments of pathologies resulting from disreactive states in human or veterinary medicine. At appropriate concentrations and in appropriate formulations, the compositions of the invention may be likewise used in cosmetics and trichological treatments.

## Claims

1. A process for preparing *in-vitro* an extract of cells of *Echinacea angustifolia* having a total titer of caffeoylquinic acids higher than 10% by weight comprising:
i) obtaining calluses from tissues of *Echinacea angustifolia* plants;
ii) stabilizing the cell line obtained from the callus tissue;
iii) selecting a cell culture with a high content of caffeoylquinic acids;
iv) transferring the stabilized and selected cell culture into liquid culture medium;
v) producing a biomass in appropriate fermenters; and
vi) extracting the cellular content of the cells of the biomass to recover said extract comprising the following stages:
- forming a homogenized suspension of said cell culture,
- eliminating particles,
- extracting the resulting solution with a solvent capable of dissolving caffeoylquinic acids, at pH values ranging between 3 and 4, or extracting the resulting solution with hydrophobic interaction resins, at pH values ranging between 3 and 4.

2. The process according to claim 1 comprising prior to the extraction step vi) the filtering of the biomass obtained from fermentation of *Echinacea angustifolia* cell cultures thus isolating cells only and discarding the medium and adding a volume of a water-soluble 2- to 5-carbon atoms alcohol together with 0.1-0.8 g/l of ascorbic acid;
And wherein in said process:
- Said stage of forming a homogenized suspension is realized by using a device capable of disgregating cells and their internal structures;
- Said stage of eliminating particles is realized by removing cell debris from the suspension by centrifugation or filtration using a filtering device with a porosity ranging between 10 and 300 µm,
- Said stage of extracting the resulting solution is realized by removing alcohol and part of water from the filtrate hydro-alcoholic phase, adjusting the suspension pH to values ranging from 3 to 4 by addition of 1.0-3.0 g/l of citric acid or another organic or mineral acid, extracting the suspension with ethyl acetate or another partially water-soluble solvent, separating and anhydrifying the organic phase and distilling the solvent of the anhydrous organic phase to give a solid compound with a total titer of caffeoylquinic acids higher than 10% by weight.

3. Process according to claim 1 wherein :
- Said stage of forming a homogenized suspension is realized by homogenizing the entire culture with a device capable of disgregating cells and their internal structures, after adjusting pH to about 8;
- Said stage of removing particles from the suspension is realized by centrifugation or filtration through a device with a porosity ranging between 10 and 300 µm;
- Said stage of extracting the resulting solution is realized by reducing the filtrate pH values to about 4 by addition of citric acid or another organic or inorganic acid, extracting the acidified solution with ethyl acetate or another partially water-soluble solvent and anhydrifying the organic phase and distilling the resulting solution at reduced pressure to give a solid compound.

4. Process according to claim 1 wherein:
- Said stage of forming a homogenized suspension is realized by homogenizing the entire culture with a device capable of disgregating cells and their internal structures, after adjusting pH to about 8;
- Said stage of removing particles from the suspension is realized by centrifugation or filtration through a device with a porosity ranging between 10 and 300 µm;
- Said stage of extracting the resulting solution is realized by reducing the filtrate pH values to about 4 by addition of citric acid or another organic or inorganic acid, extracting the acidic aqueous phase obtained with hydrophobic interaction resins, eluting said resin with an ethanol or methanol solution at a concentration ranging between 50% and 95%, distilling the eluate at reduced pressure until ethanol is completely removed and lyophilizing or extracting the aqueous residue with ethyl acetate or another appropriate reagent.

5. Process according to anyone of claims 1 to 4, using *Echinacea angustifolia* IRBEA66 selected cell culture.

6. Extract that can be obtained according to the process of anyone of claims 1 to 5, having a total titer of caffeoylquinic acids higher than 10% by weight.

7. Extract according to claim 6 having a total titer of caffeoylquinic acids ranging between 25% and 85% by weight.

8. Extract according to claim 6 or 7, containing 3-caffeoylquinic acid and one or more acids selected among 1,3-dicaffeoylquinic acid, 1,4-dicaffeoylquinic acid, 1,5-dicaffeoylquinic acid, 4,5-dicaffeoylquinic acid, 3,5-dicaffeoylquinic acid, and 1,3,5-tricaffeoylquinic acid, having a titer expressed in relation to 3-caffeoylquinic acid higher than 10% by weight.

9. Extract according to anyone of claims 6 to 8 for use as medication.

10. Extract according to claim 9, for use:
- in the prevention and treatment of acute and chronic inflammatory states;
- in a cytoprotection and detoxification treatment;
- in an hepatoprotection treatment;
- in a treatment for stimulating biliary flow through a coleretic effect;
- in the prevention and treatment of pathological disorders related to epidermal and cutaneous lesions derived from burns, incisions or lesions and mechanical traumas, diabetic sores and bedsores, thus facilitating healing and tissue repair processes;
- in an antifungal treatment;
- in a hypocholesterol treatment;
- in an antiviral treatment;
- in a neuroprotective treatment;
- in an antioxidant treatment activity in the prevention and treatment of pathological disorders related to free radical-induced damage selected from pathologies of the nervous system with an anoxic, traumatic, and inflammatory component, degenerative pathologies of the nervous system also associated with aging or with an autoimmune and inflammatory component, reperfusion damages and cardiovascular pathologies, atherosclerosis and muscular damage, dismetabolic diseases such as diabetes mellitus and the neurological, vascular, and ophthalmic complications thereof, toxic disorders such as alcoholism and the neurological complications thereof, besides hepatic cirrhosis, peripheral neuropathies including toxic neuropathies, cutaneous pathologies including cellulitis and alopecia, mucosal pathologies including oropharyngeal, gastrointestinal, and vaginal mucosae, dental pathologies, disorders of the respiratory apparatus, articular, tumoral, and ophtalmic pathologies, disorders of the auditory apparatus, and reduced immune response.

11. Pharmaceutical compositions for human or veterinary use containing an extract according to anyone of claims 6 to 8 together with pharmacologically acceptable excipients and carriers.

12. Cosmetic compositions containing an extract according to anyone of claims 6 to 8, together with cosmetically acceptable excipients and carriers.

13. Alimentary or nutraceutical compositions containing an extract according to anyone of claims 6 to 8.

14. Use of an extract according to anyone of claims 6 to 8 or a composition according to claim 12 for a cosmetic non-therapeutical cutaneaous treatment.

15. Selected cell line identified as: Echinacea angustifolia IRBEA66, deposit number DSM 17522.

## Patentansprüche

1. Verfahren zur *in vitro*-Herstellung eines Extraktes aus Zellen von *Echinacea angustifolia* mit einem Gesamttiter an Caffeoylchinasäuren von mehr als 10 Gew.-%, umfassend:
i) Erhalten von Kallussen aus Geweben von *Echinacea angustifolia-Pflanzen;*
ii) Stabilisieren der aus dem Kallusgewebe erhaltenen Zelllinie;
iii) Auswählen einer Zellkultur mit einem hohen Gehalt an Caffeoylchinasäuren;
iv) Überführen der stabilisierten und ausgewählten Zellkultur in ein flüssiges Kulturmedium;
v) Herstellen einer Biomasse in geeigneten Fermentern; und
vi) Extrahieren des Zellgehalts der Zellen der Biomasse, um den Extrakt zu wiederzugewinnen, umfassend die folgenden Stufen:
- Bilden einer homogenisierten Suspension der Zellkultur,
- Eliminieren von Partikeln,
- Extrahieren der resultierenden Lösung mit einem Lösungsmittel, das in der Lage ist, Caffeoylchinasäuren bei pH-Werten im Bereich zwischen 3 und 4 aufzulösen, oder Extrahieren der resultierenden Lösung mit hydrophoben Interaktionsharzen bei pH-Werten im Bereich zwischen 3 und 4.

2. Verfahren nach Anspruch 1, umfassend vor dem Extraktionsschritt vi) das Filtern der Biomasse, die aus der Fermentation von *Echinacea angustifolia-Zellkulturen* erhalten wurde, wodurch nur Zellen isoliert werden und das Medium entsorgt und ein Volumen eines wasserlöslichen Alkohols mit 2- bis 5-Kohlenstoffatomen zusammen mit 0,1-0,8 g/l Ascorbinsäure hinzugefügt wird;
und wobei in diesem Prozess:
- die Stufe des Bildens einer homogenisierten Suspension durch die Verwendung einer Vorrichtung realisiert wird, die in der Lage ist, Zellen und deren innere Strukturen zu zerstreuen;
- die Stufe des Eliminierens von Partikeln durch Entfernen von Zellablagerungen aus der Suspension durch Zentrifugation oder Filtration unter Verwendung einer Filtervorrichtung mit einer Porosität im Bereich zwischen 10 und 300 µm realisiert wird,
- die Stufe des Extrahierens der resultierenden Lösung durch Entfernen von Alkohol und einem Teil von Wassers aus der hydroalkoholischen Filtratphase, Einstellen des pH-Wertes der Suspension auf Werte im Bereich von 3 bis 4 durch Zugabe von 1,0-3,0 g/l Zitronensäure oder einer anderen organischen oder Mineralsäure, Extrahieren der Suspension mit Ethylacetat oder einem anderen teilweise wasserlöslichen Lösungsmittel, Trennen und Anhydrieren der organischen Phase und Destillieren des Lösungsmittels der wasserfreien organischen Phase, um eine feste Verbindung mit einem Gesamttiter an Caffeoylchinasäuren von mehr als 10 Gew.-% zu erhalten, realisiert wird.

3. Verfahren nach Anspruch 1, wobei:
- die Stufe des Bildens einer homogenisierten Suspension durch Homogenisieren der gesamten Kultur mit einer Vorrichtung realisiert wird, die in der Lage ist, Zellen und deren innere Strukturen zu zerstören, nachdem der pH-Wert auf etwa 8 eingestellt wurde;
- die Stufe des Entfernens von Partikeln aus der Suspension durch Zentrifugation oder Filtration durch eine Vorrichtung mit einer Porosität im Bereich zwischen 10 und 300 µm realisiert wird;
- die Stufe des Extrahierens der resultierenden Lösung durch Reduzieren der pH-Werte des Filtrats auf etwa 4 durch Zugabe von Zitronensäure oder einer anderen organischen oder anorganischen Säure, Extrahieren der angesäuerten Lösung mit Ethylacetat oder einem anderen teilweise wasserlöslichen Lösungsmittel und Anhydrieren der organischen Phase, und Destillieren der resultierenden Lösung bei reduziertem Druck, um eine feste Verbindung zu erhalten, realisiert wird.

4. Verfahren nach Anspruch 1, wobei:
- die Stufe des Bildens einer homogenisierten Suspension durch Homogenisieren der gesamten Kultur mit einer Vorrichtung realisiert wird, die in der Lage ist, Zellen und deren innere Strukturen zu zerstören, nachdem der pH-Wert auf etwa 8 eingestellt wurde;
- die Stufe des Entfernens von Partikeln aus der Suspension durch Zentrifugation oder Filtration durch eine Vorrichtung mit einer Porosität im Bereich zwischen 10 und 300 µm realisiert wird;
- die Stufe des Extrahierens der resultierenden Lösung durch Reduzieren der pH-Werte des Filtrats auf etwa 4 durch Zugabe von Zitronensäure oder einer anderen organischen oder anorganischen Säure, Extrahieren der mit hydrophoben Interaktionsharzen erhaltenen sauren wässrigen Phase, Eluieren des Harzes mit einer Ethanol- oder Methanollösung in einer Konzentration im Bereich zwischen 50 % und 95 %, Destillieren des Eluats bei reduziertem Druck, bis Ethanol vollständig entfernt ist, und Lyophilisieren oder Extrahieren des wässrigen Rückstands mit Ethylacetat oder einem anderen geeigneten Reagens realisiert wird.

5. Verfahren nach einem der Ansprüche 1 bis 4 unter Verwendung der ausgewählten Zellkultur *Echinacea angustifolia* IRBEA66.

6. Extrakt, der nach dem Verfahren für jeden der Ansprüche 1 bis 5 erhalten werden kann, mit einem Gesamttiter an Caffeoylchinasäuren von mehr als 10 Gew.-%.

7. Extrakt nach Anspruch 6 mit einem Gesamttiter an Caffeoylchinasäuren im Bereich zwischen 25 und 85 Gew.-%.

8. Extrakt nach Anspruch 6 oder 7, enthaltend 3-Caffeoylchinasäure und eine oder mehrere Säuren, ausgewählt aus 1,3-Dicaffeoylchinasäure, 1,4-Dicaffeoylchinasäure, 1,5-Dicaffeoylchinasäure, 4,5-Dicaffeoylchinasäure, 3,5-Dicaffeoylchinasäure und 1,3,5-Tricaffeoylchinasäure, mit einem Titer, der in Bezug auf 3-Caffeoylchinasäure höher als 10 Gew.-% exprimiert ist.

9. Extrakt nach einem der Ansprüche 6 bis 8 zur Verwendung als Medikament.

10. Extrakt nach Anspruch 9 zur Verwendung:
- bei der Prävention und Behandlung akuter und chronischer Entzündungszustände;
- bei einer Zytoprotektions- und Entgiftungsbehandlung;
- bei einer Hepatoprotektionsbehandlung;
- bei einer Behandlung zur Stimulierung des Gallenflusses durch eine kolerektische Wirkung;
- bei der Vorbeugung und Behandlung von pathologischen Störungen im Zusammenhang mit epidermalen und kutanen Läsionen, die aus Verbrennungen, Schnitten oder Läsionen und mechanischen Traumata, diabetischen Wunden und Wundliegen resultieren, um so Heilungs- und Gewebereparaturprozesse zu erleichtern;
- bei einer antimykotischen Behandlung;
- bei einer Hypocholesterinbehandlung;
- bei einer antiviralen Behandlung;
- bei einer neuroprotektiven Behandlung;
- bei einer antioxidativen Behandlungsaktivität bei der Prävention und Behandlung von pathologischen Störungen im Zusammenhang mit radikalinduzierten Schäden, ausgewählt aus Pathologien des Nervensystems mit einer anoxischen, traumatischen und entzündlichen Komponente, degenerativen Pathologien des Nervensystems, die ebenfalls mit Altern oder mit einer Autoimmun- und Entzündungskomponente verbunden sind, Reperfusionsschäden und kardiovaskulären Pathologien, Atherosklerose und Muskelschäden, dismetabolischen Erkrankungen wie Diabetes mellitus und den neurologischen, vaskulären und ophthalmischen Komplikationen davon, toxischen Störungen wie Alkoholismus und den neurologischen Komplikationen davon, daneben Leberzirrhose, peripheren Neuropathien einschließlich toxischen Neuropathien, Hautpathologien einschließlich Cellulite und Alopezie, Schleimhautpathologien, einschließlich oropharyngealen, gastrointestinalen und vaginalen Schleimhäuten, Zahnpathologien, Erkrankungen der Atemwege, artikulären, tumoralen und ophtalmischen Pathologien, Störungen des Gehörs und verminderter Immunantwort.

11. Pharmazeutische Zusammensetzungen für die menschliche oder tierärztliche Verwendung, die einen Extrakt nach einem der Ansprüche 6 bis 8 zusammen mit pharmakologisch annehmbaren Hilfsstoffen und Trägern enthalten.

12. Kosmetische Zusammensetzungen, die einen Extrakt nach einem der Ansprüche 6 bis 8 zusammen mit kosmetisch annehmbaren Hilfsstoffen und Trägern enthalten.

13. Nahrungsmittelzusammensetzungen oder nutrazeutische Zusammensetzungen, die einen Extrakt nach einem der Ansprüche 6 bis 8 enthalten.

14. Verwendung eines Extrakts nach einem der Ansprüche 6 bis 8 oder einer Zusammensetzung nach Anspruch 12 für eine kosmetische, nicht-therapeutische kutane Behandlung.

15. Ausgewählte Zelllinie, identifiziert als: Echinacea angustifolia IRBEA66, Hinterlegungsnummer DSM 17522.

## Revendications

1. Procédé de préparation *in vitro* d'un extrait de cellules d'*Echinacea angustifolia* ayant un titre total en acides caféoylquiniques supérieur à 10% en poids, comprenant :
i) obtenir des cals à partir de tissus de plantes d'*Echinacea angustifolia* ;
ii) stabiliser la lignée cellulaire obtenue à partir du tissu calleux ;
iii) sélectionner une culture cellulaire à haute teneur en acides caféoylquiniques ;
iv) transférer la culture cellulaire stabilisée et sélectionnée dans un milieu de culture liquide ;
v) produire une biomasse dans des fermenteurs appropriés ; et
vi) extraire le contenu cellulaire des cellules de la biomasse pour récupérer ledit extrait comprenant les étapes suivantes :
- former une suspension homogénéisée de ladite culture cellulaire,
- éliminer les particules,
- extraire la solution obtenue avec un solvant capable de dissoudre les acides caféoylquiniques, à des valeurs de pH comprises entre 3 et 4, ou extraire la solution obtenue avec des résines à interaction hydrophobe, à des valeurs de pH comprises entre 3 et 4.

2. Procédé selon la revendication 1, comprenant, avant l'étape d'extraction vi), la filtration de la biomasse obtenue de la fermentation de cultures de cellules d'*Echinacea angustifolia,* isolant ainsi uniquement les cellules et éliminant le milieu et ajoutant un volume d'un alcool hydrosoluble à 2-5 des atomes de carbone ensemble avec 0,1-0,8 g / 1 d'acide ascorbique ;
et dans lequel dans ledit procédé :
ladite étape de formation d'une suspension homogénéisée est réalisée en utilisant un dispositif capable de désagréger les cellules et leurs structures internes ;
ladite étape d'élimination des particules est réalisée en éliminant les débris cellulaires de la suspension par centrifugation ou filtration à l'aide d'un dispositif de filtration de porosité comprise entre 10 et 300 µm,
ladite étape d'extraction de la solution résultante est réalisée en éliminant l'alcool et une partie de l'eau de la phase hydroalcoolique du filtrat, en ajustant le pH de la suspension à des valeurs allant de 3 à 4 par addition de 1,0-3,0 g/l d'acide citrique ou d'un autre acide minéral ou organique, l'extraction de la suspension avec de l'acétate d'éthyle ou un autre solvant partiellement soluble dans l'eau, la séparation et l'anhydrification de la phase organique et la distillation du solvant de la phase organique anhydre pour donner un composé solide présentant un titre total en acide caféoylquinique supérieur à 10% en poids.

3. Procédé selon la revendication 1 dans lequel :
ladite étape de formation d'une suspension homogénéisée est réalisée en homogénéisant la totalité de la culture avec un dispositif capable de désagréger les cellules et leurs structures internes, après ajustement du pH à environ 8 ;
ladite étape d'élimination des particules de la suspension est réalisée par centrifugation ou filtration à travers un appareil de porosité comprise entre 10 et 300 µm ;
ladite étape d'extraction de la solution résultante est réalisée en réduisant les valeurs du pH du filtrat à environ 4 en ajoutant de l'acide citrique ou un autre acide organique ou inorganique, en extrayant la solution acidifiée avec de l'acétate d'éthyle ou un autre solvant partiellement soluble dans l'eau et en déshydratant la phase organique et en distillant la solution résultante à pression réduite pour donner un composé solide.

4. Procédé selon la revendication 1 dans lequel :
ladite étape de formation d'une suspension homogénéisée est réalisée en homogénéisant la totalité de la culture avec un dispositif capable de désagréger les cellules et leurs structures internes, après ajustement du pH à environ 8 ;
ladite étape d'élimination des particules de la suspension est réalisée par centrifugation ou filtration à travers un dispositif de porosité comprise entre 10 et 300 µm ;
ladite étape d'extraction de la solution résultante est réalisée en réduisant les valeurs de pH du filtrat à environ 4 par addition d'acide citrique ou d'un autre acide organique ou inorganique, en extrayant la phase aqueuse acide obtenue avec des résines à interaction hydrophobe, en éluant ladite résine avec une solution d'éthanol ou de méthanol à une concentration comprise entre 50% et 95%, en distillant l'éluat sous pression réduite jusqu'à élimination complète de l'éthanol et en lyophilisant ou extrayant le résidu aqueux avec de l'acétate d'éthyle ou un autre réactif approprié.

5. Procédé selon l'une quelconque des revendications 1 à 4, utilisant une culture de cellules sélectionnées IRBEA66 d'*Echinacea angustifolia.*

6. Extrait susceptible d'être obtenu selon le procédé de l'une quelconque des revendications 1 à 5, ayant un titre total en acides caféoylquiniques supérieur à 10% en poids.

7. Extrait selon la revendication 6, ayant un titre total en acides caféoylquiniques compris entre 25% et 85% en poids.

8. Extrait selon la revendication 6 ou 7, contenant de l'acide 3-caféoylquinique et un ou plusieurs acides choisis parmi l'acide 1,3-dicaffeoylquinique, l'acide 1,4-dicaffeoylquinique, l'acide 1,5-dicaffeoylquinique, l'acide 4,5-dicaffeoylquinique, l'acide 3,5-dicaffeoylquinique et l'acide 1,3,5-tricaffeoylquinique, ayant un titre exprimé en acide 3-caffeoylquinique supérieur à 10% en poids.

9. Extrait selon l'une quelconque des revendications 6 à 8 pour une utilisation en tant que médicament.

10. Extrait selon la revendication 9, pour une utilisation :
- dans la prévention et le traitement des états inflammatoires aigus et chroniques ;
- dans un traitement de cytoprotection et de détoxification ;
- dans un traitement d'hépatoprotection ;
- dans un traitement stimulant la circulation biliaire par effet colérétique ;
- dans la prévention et le traitement des troubles pathologiques liés aux lésions épidermiques et cutanées résultant de brûlures, incisions ou lésions et traumatismes mécaniques, de plaies diabétiques et escarres, facilitant ainsi les processus de guérison et de réparation tissulaire ;
- dans un traitement antifongique ;
- dans un traitement à l'hypocholestérol ;
- dans un traitement antiviral ;
- dans un traitement neuroprotecteur ;
- dans une activité de traitement antioxydant dans la prévention et le traitement des désordres pathologiques liés aux dommages induits par les radicaux libres choisis parmi les pathologies du système nerveux à composante anoxique, traumatique et inflammatoire, les pathologies dégénératives du système nerveux également associées au vieillissement ou à une composante auto-immunitaire et inflammatoire, les dommages de reperfusion et les pathologies cardiovasculaires, l'athérosclérose et les dommages musculaires, les maladies métaboliques telles que le diabète sucré et ses complications neurologiques, vasculaires et ophtalmiques, les troubles toxiques tels que l'alcoolisme et ses complications neurologiques, en plus de la cirrhose hépatique périphérique, les neuropathies comprenant des neuropathies toxiques, les pathologies cutanées comprenant la cellulite et l'alopécie, des pathologies de la muqueuse incluant la muqueuse oropharyngée, gastro-intestinale et vaginale, les pathologies dentaires, les troubles de l'appareil respiratoire, les pathologies articulaires, tumorales et ophtalmiques, les troubles de l'appareil auditif, et une réponse immunitaire réduite.

11. Compositions pharmaceutiques à usage humain ou vétérinaire contenant un extrait selon l'une quelconque des revendications 6 à 8, avec des excipients et des véhicules pharmacologiquement acceptables.

12. Compositions cosmétiques contenant un extrait selon l'une quelconque des revendications 6 à 8, ainsi que des excipients et des véhicules cosmétiquement acceptables.

13. Compositions alimentaires ou nutraceutiques contenant un extrait selon l'une quelconque des revendications 6 à 8.

14. Utilisation d'un extrait selon l'une quelconque des revendications 6 à 8 ou d'une composition selon la revendication 12 pour un traitement cutané cosmétique non-thérapeutique.

15. Lignée cellulaire sélectionnée identifiée comme : *Echinacea angustifolia* IRBEA66, numéro de dépôt DSM 17522.
